(19) Europäisches Patentamt
European Patent Office
Office européen des brevets

(11) Publication number: **0 426 460 A1**

(12) # EUROPEAN PATENT APPLICATION

(21) Application number: 90311946.9

(22) Date of filing: 31.10.90

(51) Int. Cl.[5]: C07C 251/60, C07C 323/47, A01N 37/36

(30) Priority: 02.11.89 JP 284754/89
02.11.89 JP 284755/89
27.02.90 JP 44622/90
02.03.90 JP 49340/90

(43) Date of publication of application:
08.05.91 Bulletin 91/19

(84) Designated Contracting States:
CH DE ES FR GB IT LI

(71) Applicant: UBE INDUSTRIES, LTD.
12-32, Nishihonmachi 1-chome
Ube-shi, Yamaguchi-ken(JP)

(72) Inventor: Watanabe, Masanori, c/o Ube Research Laboratory
Ube Industries Ltd., 1978-5, Oaza Kogushi
Ube-shi, Yamaguchi-ken(JP)
Inventor: Tanaka, Toshinobu, c/o Ube Research Laboratory
Ube Industries Ltd., 1978-5, Oaza Kogushi
Ube-shi, Yamaguchi-ken(JP)
Inventor: Kobayashi, Hisato, c/o Ube Research Laboratory
Ube Industries Ltd., 1978-5, Oaza Kogushi
Ube-shi, Yamaguchi-ken(JP)
Inventor: Yokoyama, Shuji, c/o Ube Research Laboratory
Ube Industries Ltd., 1978-5, Oaza Kogushi
Ube-shi, Yamaguchi-ken(JP)

(74) Representative: Green, Mark Charles et al
Urquhart-Dykes & Lord, 91 Wimpole Street
London W1M 8AH(GB)

(54) **Oxime ether derivative, preparation thereof and germicide containing the same.**

(57) Disclosed are a compound represented by the formula (I):

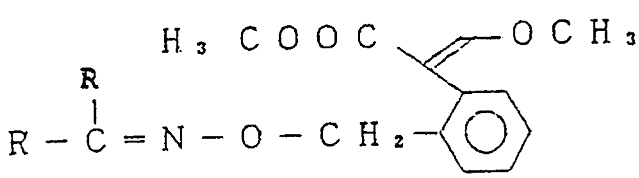

(I)

wherein Q and R are described in the specification, a method for preparing the compound of the formula (I) by reacting a compound represented by the formula (II):

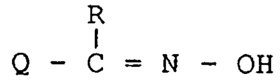

(II)

wherein Q and R are the same as defined above, with a compound of the formula (III):

EP 0 426 460 A1

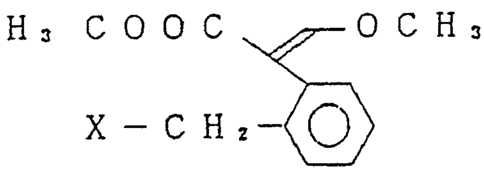

(III)

wherein X represents an eliminable group, under alkaline conditions, and a germicide containing the compound of the formula (I) as an active ingredient.

# OXIME ETHER DERIVATIVE, PREPARATION THEREOF AND GERMICIDE CONTAINING THE SAME

## BACKGROUND OF THE INVENTION

This invention relates to an oxime ether derivative, preparation thereof and a germicide or fungicide containing the same as an active ingredient.

Among oxime ether derivatives, for example, compounds disclosed in (1) Japanese Unexamined Patent Publication No. 258166/1985, (2) Japanese Unexamined Patent Publication No. 114971/1987, (3) Japanese Unexamined Patent Publication No. 283962/1987, and (4) Cruzate (trade name: Du Pont) have been known to have germicidal activities.

However, the compounds disclosed in (1) to (3) have no germicidal effect to the extent which can sufficiently inhibit onset of diseases, and further no germicidal effect can be expected at low concentration, while Cruzate of (4) has substantially no preventive effect recognized so as to make plants grow healthily without onset of diseases.

## SUMMARY OF THE INVENTION

An object of the present invention is to provide a novel oxime ether derivative, a method for preparation thereof and a germicide containing the same as the active ingredient.

The present inventors have studied intensively in order to solve the above-mentioned problems, and consequently found that a novel oxime ether derivative has potent germicidal activity even at low concentration and therefore has excellent preventive and therapeutical effect against plant pathogens to accomplish the present invention.

More specifically, the present invention concerns: A compound represented by the formula (I):

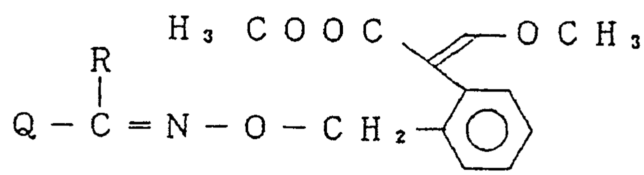

(I)

wherein R represents at least one selected from the group consisting of an alkylthio group having 1 to 10 carbon atoms, a halo-lower alkyl group and cyano group;
when R is an alkylthio group having 1 to 10 carbon atoms or a halo-lower alkyl group, Q represents at least one selected from the group consisting of a phenyl group substituted with an alkyl group having 1 to 6 carbon atoms, a halogen atom, nitro group, an alkoxy group having 1 to 4 carbon atoms, a phenoxy group which may be substituted, methylenedioxy group, cyano group or a halo-lower alkyl group, pyridyl group which may be substituted, a naphthyl group, a hetoroaryl group, cinnamyl group which may be substituted, and a cycloalkyl group having 3 to 6 carbon atoms; and
when R is cyano group, Q represents at least one selected from the group consisting of a phenyl group which may be substituted with an alkyl group having 1 to 6 carbon atoms, a halogen atom, nitro group, an alkoxy group having 1 to 4 carbon atoms, a phenoxy group which may be substituted, methylenedioxy group, cyano group or a halo-lower alkyl group, a naphthyl group, cinnamyl group which may be substituted, and a cycloalkyl group having 3 to 6 carbon atoms.

A method for preparing a compound of the above formula (I), which comprises reacting a compound of the formula:

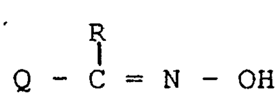

(II)

wherein Q and R are the same as defined above, with a compound of the formula:

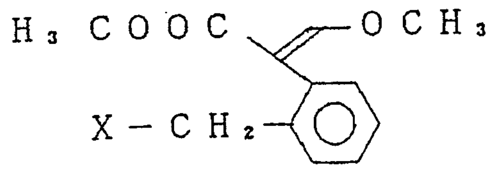

(III)

wherein X represents an eliminable group, under alkaline conditions; and
A germicide comprising a compound of the above formula (I) as the active ingredient.

## DESCRIPTION OF THE PREFERRED EMBODIMENTS

In the novel oxime ether derivative (I) and the compounds of (II) and (III) which are it's starting materials, Q may include a phenyl group which may be substituted with an alkyl group having 1 to 6 carbon atoms, a halogen atom such as chlorine atom, fluorine atom, bromine atom, iodine atom, etc., nitro group, an alkoxy group having 1 to 4 carbon atoms such as methoxy group, ethoxy group, propoxy group, butoxy group, etc., methylenedioxy group, cyano group, or halo-lower alkyl group such as an alkyl group substituted with 1 to 3 halogen atoms including, for example, chloromethyl group, fluoromethyl group, trifluoromethyl group, 1- or 2-chloroethyl group, 1- or 2-fluoroethyl group, etc.; pyridyl group which may be substituted; naphthyl group; a heteroaryl group such as furan, thiophene, pyridine and benzo derivatives thereof; cinnamyl group which may be substituted; a heteroaryl group; a cycloalkyl group having 3 to 6 carbon atoms such as cyclopropyl group, cyclopentyl group, cyclohexyl group, etc.

However, when R is cyano group as mentioned below, the pyridyl group which may be substituted and the heteroaryl group are excluded from the scope of the present invention, and when R is an alkylthio group having 1 to 10 carbon atoms or a halo-lower alkyl group, unsubstituted phenyl group is excluded.

The substituent in the phenyl group which may be substituted may include, as an alkyl group having 1 to 6 carbon atoms, stranght- or branched chain alkyl group having 1 to 6 carbon atoms, preferably those having 1 to 4 carbon atoms, more preferably methyl group, ethyl group, t-butyl group, etc. The substitution position of the nitro group is preferably 2-, 3- or 4-position. As the halogen atom, preferred is chlorine atom, bromine atom or iodine atom, more preferably the substitution position thereof is 2-, 3- or 4-position. As the alkoxy group having 1 to 4 carbon atoms, included as an alkoxy group having 1 to 4 carbon atoms are a straight- or branched-chain alkoxy group having 1 to 4 carbon atoms, preferably those having 1 to 3 carbon atoms, further preferably methoxy group, most preferably the substitution position thereof is 2-position. The halo-lower alkyl group may include preferably trifluoromethyl group, further preferably the substitution position thereof is 4-position. The substitution position of the cyano group is 3- or 4-position.

As the heteroaryl group, preferred is thiophene group. As the cycloalkyl group having 3 to 6 carbon atoms, preferred is a cyclopropyl group.

R may include an alkylthio group having 1 to 10 carbon atoms and a halo-lower alkyl group such as an alkyl group having 1 to 2 carbon atoms substituted with 1 to 3 halogen atoms including, for example, chloromethyl group, fluoromethyl group, trifluoromethyl group, 1- or 2-chloroethyl group, 1- or 2-fluoroethyl group, etc. The halo-lower alkyl group may include trifluoromethyl group. The alkyl group in the alkylthio group having 1 to 10 carbon atoms may include a straight- or branched-chain alkyl group, preferably those having 1 to 8 carbon atoms.

When R is an alkylthio group, Q is preferably a phenyl group substituted with at least one selected from the group consisting of mono halogen atom, mono alkyl group having 1 to 6 carbon atoms and a branched alkyl group having 1 to 6 carbon atoms. From another view, when R is an alkylthio group, Q is preferably a phenyl group substituted with a mono group.

When R is cyano group, Q is preferably a phenyl group substituted with an alkyl group having 1 to 6 carbon atoms, a halogen atom, nitro group, an alkoxy group having 1 to 4 carbon atoms, a phenoxy group which may be substituted, methylenedioxy group, cyano group or a halo-lower alkyl group, more preferably a is a phenyl group substituted with an alkyl group having 1 to 6 carbon atoms at 4-position or a halogen atom, further preferably Q is 3- or 4-trifluoromethylphenyl.

X is an eliminable group and not particularly limited. may include, for example, a halogen atom such as chlorine, bromine, iodine, etc., an alkanesulfonyloxy group which may be substituted with a halogen atom such as methanesulfonyloxy, ethanesulfonyloxy, trifluoromethanesulfonyloxy, etc., an arylsulfonyloxy group

such as benzenesulfonyloxy, p-toluenesulfonyloxy, etc., preferably a halogen atom, further preferably bromine atom.

The desired compound (I), since it has two double bonds of one >C=N- and one >C=C< in combination, is obtained as a mixture of geometric isomers, and can be separated into individual isomers, if necessary.

As preferable examples of the desired compound (I), the compounds represented by the following formula can be included:

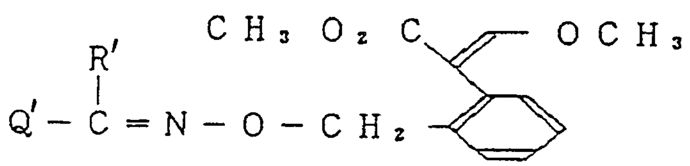

(I-A)

(wherein Q′ represents a phenyl group substituted with a halogen atom, nitro group or an alkoxy group, naphthyl group, thiophen or a cyclopropyl group; R′ represents trifluoromethyl group). More preferable desired compound (I) is exemplified in the following Table 1.

Table 1

| No. | Q' | R' | Physical properties |
|---|---|---|---|
| 1 | 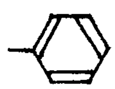 | $CF_3$ | $n_D^{24}$ 1.5386 |

As other preferable examples of the desired compound (I), there may be included the compounds of the formula (I-B):

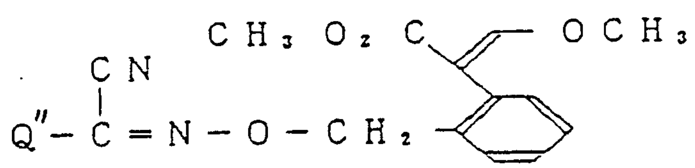

(I-B)

(wherein Q″ represents a phenyl group which may be substituted with an alkyl group having 1 to 6 carbon atoms, a halogen atom, nitro group, an alkoxy group having 1 to 4 carbon atoms or phenoxy group, and as more preferable desired compounds, those as exemplified in the following Table 2 can be included.

Table 2

| No. | Q" | Physical properties |
|---|---|---|
| 2 | Phenyl | m.p. 99 to 102 °C |
| 3 | 4-Methylphenyl | $n_D^{27.6}$ 1.5462 |
| 4 | 4-Chlorophenyl | $n_D^{24.6}$ 1.5798 |
| 5 | 2-Chlorophenyl | m.p. 88 to 84 °C |
| 6 | 4-Bromophenyl | NMR spectrum (a) |
| 7 | 4-Nitrophenyl | m.p. 98 to 102 °C |
| 8 | 2-Nitrophenyl | $n_D^{22.0}$ 1.5706 |
| 9 | 2-Methoxyphenyl | $n_D^{26.0}$ 1.5714 |
| 10 | 3-Phenoxyphenyl | NMR spectrum (b) |
| 11 | 3,4-Methylenedioxyphenyl | m.p. 114 to 118 °C |

(a) $^1$NMR($CDCl_3$) δ ppm
3.70 (3H,s), 3.82 (3H,s), 5.31 (2H,s), 7.01 - 7.63 (9H)

(b) $^1$NMR($CDCl_3$) δ ppm
3.70 (3H,s), 3.82 (3H,s), 5.31 (2H,s), 7.01 - 7.63 (14H)

As preferable examples of the starting compound (II), when the starting compound (I) is the compound of the formula (I-A), compounds of the formula:

$$Q' - \overset{\overset{\displaystyle R'}{|}}{C} = N - OH \qquad (II\text{-}A)$$

(wherein Q′ and R′ are the same as in (I-A)) can be included, or when the starting compound (I) is the compound of the formula (I-B), compounds of the formula:

$$Q'' - \overset{\overset{\displaystyle CN}{|}}{C} = N - OH \qquad (II\text{-}B)$$

(wherein Q″ is the same as in (I-B)) can be included.

As preferable examples of the starting compound (III), compounds of the formula:

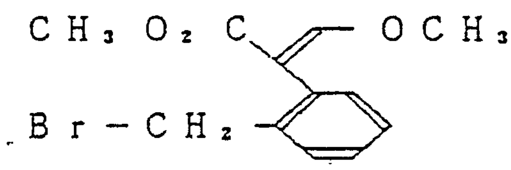

(III-A)

can be included.

The starting compound (II) to be used in the present invention can be easily prepared according to, for example, the method as described in Shinjikken Kagaku Koza (New Experimental Chemical Course), Vol. 14, III, p. 1301 (Maruzen), by reacting a ketone with hydroxylamine hydrochloride in the presence of an appropriate base such as potassium acetate, by use of an appropriate solvent such as toluene, ethanol, methylene chloride, water, if necessary under heating.

The starting compound (II) to be used in the present invention, when R is cyano group, can be easily prepared according to, for example, the method as described in Organic Reaction Vol. 7, p. 327, John Wiley & Sons, INC. (1959) by treating a compound of the formula:

$Q' - CH_2CN$

[wherein Q′ is the same as in (I-A)]

with an appropriate alkyl nitrite such as methyl nitrite, isoamyl nitrite, etc. in the presence of an appropriate acid such as hydrogen chloride or an appropriate base such as sodium ethylate.

The starting compound (III) to be used in the present invention can be easily prepared according to, for example, the method as described in Japanese Unexamined Patent Publication No. 280452/1986, by treating methyl-2-methoxy-1-(2-methylphenyl)-acrylate with a halogenating agent such as N-bromosuccinimide.

The method for preparing the desired compound (I) can be practiced by reacting the starting compound (II) with the starting compound (III) in an organic solvent in the presence of a base.

Examples of the organic solvent which can be used in the preparation method may include preferably aprotic solvents such as acetonitrile, dimethylformamide, dimethyl sulfoxide, etc.; ether solvents such as tetrahydrofuran, diethyl ether, dioxane, etc.; polar solvents such as acetone, etc., further preferably an aprotic solvent such as acetonitrile, etc. may be used.

As the base, for example, potassium carbonate, sodium carbonate and sodium hydride can be included, but it is preferable to employ potassium carbonate.

The preparation method of the desired compound (I) can be practiced at a reaction concentration of 0.1 to 40 %.

In the preparation method, the ratio of the starting compound (II) to the starting compound (III) employed can be 0.1 to 4 moles, preferably 0.5 to 1.5 moles, of the starting compound (III) to one mole of the starting compound (II).

The reaction temperature in the preparation method may be -10 to 130 °C, preferably 10 to 80 °C.

The preparation method can be practiced generally within 0.5 to 8 hours, although it varies depending on the above-mentioned concentration and temperature.

Isolation of the desired compound (I) can be practiced by obtaining simply the crude desired compound (I) of the present invention by removing the solvent from the reaction mixture after completion of the reaction, and purifying further the crude compound by conventional purification methods such as recrystallization, chromatography, etc. to give the desired compound (I).

The compound of the present invention can be used for prevention and therapy of plant diseases such as downy mildew and blight of vegetables, grape downy mildew, blast, wheat red mould, powdery mildew, scab, leaf-sheath disease, etc., but has marked germicidal effect for prevention and therapy of cucumber downy mildew, rice blast, barley powdery mildew and wheat red mould.

The germicide of the present invention contains at least one of the compounds (I) as the active ingredient.

The compound (I) can be also used alone, but usually preferably employed by formulating in conventional manner carriers, surfactants, dispersing agents or auxiliary agents (for example, prepared with a composition of powder, emulsion, fine granules, granules, wettable agent or oily suspension, aerosol, etc.).

As the carrier, there may be included, for example, solid carriers such as talc, bentonite, clay, kaolin, diatomaceous earth, white carbon, vermicullite, slake lime, siliceous sand, ammonium sulfate, urea, etc.; liquid carriers, for example, hydrocarbons such as kerosine, mineral oils, etc.; aromatic hydrocarbons such as benzene, toluene, xylene, etc.; chlorinated hydrocarbons such as chloroform, carbon tetrachloride, etc.;

ethers such as dioxane, tetrahydrofuran, etc.; ketones such as acetone, cyclohexanone, isophorone, etc.; esters such as ethyl acetate, ethylene glycol acetate, dibutyl maleate, etc.; alcohols such as methanol, n-hexanol, ethylene glycol, etc.; polar solvents such as dimethyl formamide, dimethyl sulfoxide, etc. or water; gaseous carriers such as air, nitrogen, carbon dioxide, Freon (in this case, can be jetted as a mixture), etc.

As the surfactant or the dispersing agent which can be used for improving attachment, adsorption of the present agent onto animals and plants, improving the performances such as dispersion, emulsification, spreading of the chemical, there can be included, for example, alcohol sulfuric acid esters, alkylsulfonic acid salts, lignin-sulfonic acid salts, polyoxyethylene glycol ethers, etc. And, for improvement of the properties of the preparation, carboxymethyl cellulose, polyethylene glycol, gum arabic, etc. can be used as the auxiliary agent.

In preparation of the present agent, the above-mentioned carrier, surfactant, dispersing agent and auxiliary agent can be used each individually or in a suitable combination depending on the respective purposes.

The active ingredient concentration when the compound (I) of the present invention is formed into a preparation may be generally 1 to 50 % by weight for emulsion, generally 0.3 to 25 % by weight for powder, generally 1 to 90 % by weight for wettable agent, generally 0.5 to 5 % by weight for granules, generally 0.5 to 5 % by weight for oil agent and generally 0.1 to 5 % by weight for aerosol.

These preparations can be diluted to appropriate concentrations, and provided for various uses by spraying them on plant stalks and leaves, soil, water surface of paddy field, or applying them directly depending on the respective purposes.

The present invention is described below by referring to Reference examples and Examples, but these examples are not intended to limit the scope of the invention.

Reference example 1

[Synthesis of starting compound (II)]

7 g of 2-fluorobenzaldoxime was dissolved in 250 ml of chloroform and to the solution were brown chlorine gas at 0 °C for 15 minutes. After excess chlorine was decomposed with 1N-sodium thiosulfate solution, the resultant was washed with water, dried and then concentrated. The residue was dissolved in 20 ml of THF, and to the, solution was added 25 ml of 15 % sodium methyl mercaptan solution at room temperature. After stirring for 1 hour, the resulting solution was extracted with ether, washed with water, dried and then concentrated, followed by purification with column chromatography to obtain 6.8 g of α-methylthio-2-fluorobenzaldoxime as colorless oily products.

Reference example 2

[Synthesis of starting compound (III)]

An amount 8.83 g of methyl-1-(2-methylphenyl)-2-methoxyacrylate and 7.6 g of N-bromosuccinimide were dissolved in ml of carbon tetrachloride, and 0.3 g of benzoyl peroxide was added, followed by heating under reflux for 4 hours. The resultant was cooled to room temperature, filtrated, concentrated and crystallized from n-hexane to give 11.6 g of α-(2-bromomethylphenyl)-β-methoxyacrylate as colorless crystals. (m.p. 85 - 87 °C)

Reference example 3

[Synthesis of starting compound (II)]

4 g of p-Methylphenylacetonitrile and 10 ml of i-amyl nitrite were dissolved in 30 ml of ethanol, and 2.2 g of sodium ethoxide was added thereto, followed by stirring at room tempera-ture for 8 hours.

To the mixture was added 50 ml of water, and the mixture was extracted with ether, dried over

anhydrous magnesium sulfate, then the solvent was removed, and the residue was purified by silica gel column chromatography (Wako gel C-200, toluene:ethyl acetate = 5:1) to give 2.7 g of α-oximino-p-methylphenylacetonitrile as colorless crystals.

## Example 1

[Synthesis of 3-methoxy-2-(methylthio(2-fluorophenyl)}methyliminoxymethylphenylacrylate]

To a mixture of 0.56 g of α-methylthio-2-fluorobenzaldoxime, 0.41 g of potassium carbonate and 10 ml of acetonitrile was added 0.57 g of methyl-3-methoxy-2-(2-bromomethylphenyl)acrylate, and the mixture was stirred at 50 °C for 3 hours. After being cooled to room temperature, the resultant was added with 10 ml of water and extracted with ethyl acetate. After drying, it was concentrated, followed by purification of the residue with column chromatography to obtain 0.70 g of 3-methoxy-2-{methylthio(2-fluorophenyl)-}methyliminoxymethylphenylacrylate as colorless oily products.

## Examples 2

By use of the starting compound (III) of Reference 2 and by use of the starting compound (II) as shown in Table 3 prepared by use of other compound in place of 2-fluorobenzaldoxime in Reference example 1, the desired compound (I) could be obtained according to the same method as in Example 1.

Table 3

Compound (II)

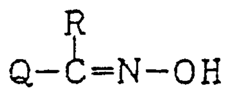

Compound (III)

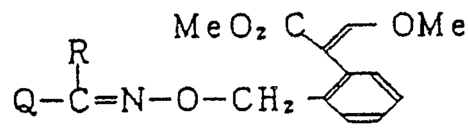

| Compound No. | Q | R | Physical properties |
| --- | --- | --- | --- |
| 1 | 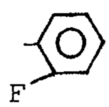 | SMe | $n_D^{21}$ 1.5795 |
| 2 | 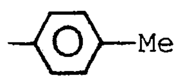 | SMe | $n_D^{21}$ 1.5940 |
| 3 | 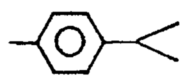 | SMe | $n_D^{21}$ 1.5860 |
| 4 | 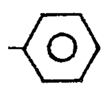 | $S-C_3H_{7}-n$ | $n_D^{26}$ 1.5838 |
| 5 | 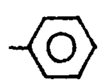 | $S-C_8H_{17}-n$ | $n_D^{26}$ 1.5537 |

(Me represents $CH_3$ group)

Example 3

[Preparation of wettable agent of fine powder]

An amount 10 parts by weight of the compound of Example 1, 69.75 parts by weight of kaolin, 18 parts by weight of white carbon, 1.8 parts by weight of Neopelex powder (trade name: Kao-Atlas) and 0.45 part by weight of Demol EP (trade name: Kao-Atlas) were uniformly mixed and pulverized to obtain a wettable agent of fine powder.

Example 4

[Preparation of emulsion]

An amount 50 parts by weight of the compound of Example 2, parts by weight of xylene, 10 parts by weight of Agrisol P-300 (trade name: Kao-Atlas), 5 parts by weight of Emulgen A-90 (trade name: Kao-Atlas) and 5 parts by weight of Leosol 460 (trade name: Kao-Atlas) were mixed and dissolved to obtain an emulsion.

Example 5

[Synthesis of methyl-α-methoxymethylene-o-(p-methylphenyl)cyanomethaneiminooxymethylphenyl acetate (Compound No. 6)]

0.57 g (2 mmol) of Methyl-2-[2-(bromomethyl)phenyl]-3-methoxypropenoate and 0.33 g (2.06 mmol) of α-oxyimino-(4-methylphenyl)-acetonitrile were dissolved in 15 ml of acetonitrile, and to the solution was added 0.33 g (2.4 mmol) of potassium carbonate and the mixture was heated at 60 °C for 4 hours. The mixture was cooled to room temperature, and 50 ml of water was added, followed by extraction with ether. The organic solvent layer was washed with saturated aqueous sodium chloride, dried over anhydrous magnesium sulfate and then the solvent was evaporated. The residue obtained was isolated by silica gel column chromatography (Wako Gel C-200, toluene:ethyl acetate = 5:1) to obtain 0.6 g of amber desired product No. 6 (yield: 83 %)

Examples 6

By use of the starting compound (III) of Reference example 2 and the starting compound (II) as shown in Table 4 prepared by use of other compounds in place of p-chloroacetophenone in Reference example 3, the desired compound (I) (Nos. 7 to 31) could be obtained according to the same method as in Example 5.

Table 4

Compound (II)

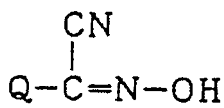

Compound (III)

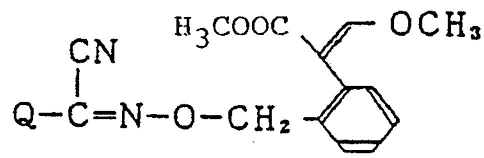

| Compound No. | Q | Yield (%) | Physical properties |
|---|---|---|---|
| 6 | Phenyl | 83 | m.p. 99 - 102 °C |
| 7 | 4-Methylphenyl | 68 | $n_D^{27.6}$ 1.5462 |
| 8 | 4-Chlorophenyl | 87 | $n_D^{24.6}$ 1.5798 |
| 9 | 2-Chlorophenyl | 82 | m.p. 82 - 84 °C |
| 10 | 4-Bromophenyl | 85 | NMR spectrum (a) |
| 11 | 4-Nitrophenyl | 90 | m.p. 98 - 102 °C |
| 12 | 2-Nitrophenyl | 82 | $n_D^{22.0}$ 1.5706 |
| 13 | 2-Methoxyphenyl | 71 | $n_D^{26.0}$ 1.5714 |
| 14 | 3-Phenoxyphenyl | 62 | NMR spectrum (b) |

| | | | |
|---|---|---|---|
| 15 | 3,4-Methylenedioxy-phenyl | 76 | m.p. 114 - 118 °C |
| 16 | 4-Cyanophenyl | | $n_D^{26.0}$ 1.5625 |
| 17 | 2,4-Dichlorophenyl | | $n_D^{26.0}$ 1.5606 |
| 18 | p-Ethylphenyl | | $n_D^{23.0}$ 1.5775 |
| 19 | 3,5-Dichlorophenyl | | m.p. 52 - 53 °C |
| 20 | 4-Methyl-3-nitrophenyl | | NMR spectrum (c) |
| 21 | 2-Chloro-3-nitrophenyl | | NMR spectrum (d) |
| 22 | 3-Iodophenyl | | $n_D^{23.0}$ 1.5976 |
| 23 | 3-Cyanophenyl | | $n_D^{23.0}$ 1.5640 |
| 24 | 3-Nitrophenyl | | m.p. 82 - 84 °C |
| 25 | 4-Trifluoromethylphenyl | | m.p. 88 - 89 °C |
| 26 | 4-t-Butylphenyl | | $n_D^{25.0}$ 1.5558 |
| 27 | 3-Trifluoromethylphenyl | | $n_D^{25.0}$ 1.5478 |
| 28 | 3-Methylphenyl | | NMR spectrum (e) |
| 29 | 4-isopropylphenyl | | $n_D^{25.0}$ 1.5636 |
| 30 | 4-isobutylphenyl | | $n_D^{25.0}$ 1.5568 |
| 31 | 3,4-Dichlorophenyl | | $n_D^{25.0}$ 1.5780 |

(a) $^1$NMR($CDCl_3$) δ ppm

3.70 (3H,s), 3.82 (3H,s), 5.31 (2H,s), 7.01 - 7.63 (9H)

(b) $^1$NMR($CDCl_3$) δ ppm

3.70 (3H,s), 3.82 (3H,s), 5.30 (2H,s), 7.00 - 7.62 (14H)

(c) $^1$NMR($CDCl_3$) δ ppm

2.63 (3H,s), 3.72 (3H,s), 3.85 (3H,s), 5.36 (2H,s), 7.18 - 7.64 (8H)

(d) $^1NMR(CDCl_3)$ δ ppm

3.71 (3H,s), 3.83 (3H,s), 5.32 (2H,s), 7.14 - 7.64 (8H)

(e) $^1NMR(CDCl_3)$ δ ppm

2.40 (3H,s), 3.70 (3H,s), 3.82 (3H,s), 5.32 (3H,s), 7.14 - 7.62 (9H)

Example 7

[Preparation of emulsion]

An amount 50 parts by weight of Compound No. 6 of the present invention, 30 parts by weight of xylene, 10 parts by weight of Agrisol P-300 (trade name: Kao-Atlas), 5 parts by weight of Emulgen A-90 (trade name: Kao-Atlas) and 5 parts by weight of Leosol 460 (trade name: Kao-Atlas) were uniformly mixed to obtain an emulsion.

Example 8

[Preparation of wettable agent of fine powder]

An amount 10 parts by weight of Compound No. 7 of the present invention, 69.75 parts by weight of kaolin, 18 parts by weight of white carbon, 1.8 parts by weight of Neopelex powder (trade name: Kao-Atlas) and 0.45 part by weight of Demol EP (trade name: Kao-Atlas) were uniformly mixed and pulverised to obtain a wettable agent of fine powder.

Example 9

[Control activity test against cucumber downy mildew (preventive effect)]

One cucumber (species: Sagami Hanpaku) per one pot was grown in a plastic planting pot of 6 cm in diameter, and onto the young plant of 1.5 leaf stage was sprayed the desired compound (I) shown in Table 3 and Table 4, prepared into the wettable agent according to Example 3 or 8, diluted to 50 ppm with a water containing a surfactant (0.01 %) in each 20 ml per one pot. After spraying, a suspension of spores (7 x $10^4$ spores/ml) of cucumber downy mildew microorganism (Pseudoperonospora cubemsis ) was uniformly sprayed and inoculated on the back of the plant leaf.

After inoculation, the plant was kept at 20 °C in the dark for one day, and then grown in a glass greenhouse for 5 days, and the extent of the cucumber downy mildew lesion appeared at the first leaf was examined.

Judgement of the chemical effect was compared with the extent of the lesion of the non-treated group.

Evaluation is shown at 6 ranks of 5, 4, 3, 2, 1, 0, with one having no lesion being represented as 5, one with lesion area as compared with non-treated group of 10 % or less as 4, one about 20 % as 3, one about 40 % as 2, one about 60 % as 1, and one with the whole being afflicted as 0. As the Control compound, 4-(4-chlorobenzoyloxyimino)-1,3-dimethyl-2-pyrazoline-5-one represented by the following formula (C) was employed at 250 ppm.

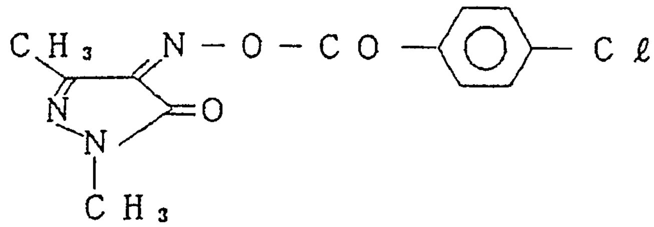

(C)

The results are shown in Table 5.

Table 5

| Compound No. | Preventive effect against cucumber downy mildew |
|---|---|
| 1 | 5 |
| 2 | 5 |
| 3 | 5 |
| 6 | 5 |
| 7 | 5 |
| 8 | 5 |
| 9 | 5 |
| 10 | 5 |
| 13 | 5 |
| 16 | 4 |
| 17 | 4 |
| 18 | 5 |
| 19 | 4 |
| 22 | 5 |
| 24 | 5 |
| 25 | 5 |
| 26 | 5 |
| 28 | 5 |
| 29 | 5 |
| 30 | 4 |
| 31 | 5 |
| C | 1 |

Example 10

[Control effect test against rice blast (preventive effect)]

In a plastic planting pot of 6 cm in diameter were grown rice seedlings (species: Nipponbare) per one pot, and onto the young plant of 1.5 leaf stage was sprayed the desired compound (I) shown in Table 3 and 4, prepared into the wettable agent according to Example 3 or 8, diluted to 200 ppm with a water containing a surfactant (0.01 %) in an amount of each 20 ml per one pot.

After spraying, the rice was cultivated in a glass greenhouse for 2 days, and then a suspension of mycelial spores (7 x $10^4$ spores/ml) of rice blast microorganism (Pyricularia oryzae ) was sprayed and inoculated uniformly on the plant leaves.

After inoculation, the plants were grown in a wet chamber at 28 °C for 5 days, and the extent of the rice blast lesion appearing on the leaves was examined.

Judgement of the chemical effect was compared with the extent of the lesion of the non-treated group.

The results are shown in Table 6 according to the same evaluation method as in Example 9. As Control

compound, the compound (C) shown in Example 9 was employed at 200 ppm.

Table 6

| Compound No. | Preventive effect against rice blast |
|---|---|
| 3 | 5 |
| 6 | 5 |
| 7 | 5 |
| 8 | 5 |
| 9 | 5 |
| 10 | 5 |
| 11 | 5 |
| 13 | 5 |
| 27 | 5 |
| 28 | 5 |
| 29 | 5 |
| 30* | 5 |
| 31 | 5 |
| C | 0 |

(* diluted to 500 ppm)

Example 11

[Control activity test against barley powdery mildew (preventive effect)]

In a plastic planting pot of 6 cm in diameter, 10 barleys (species: Kuromugi) were grown per one pot, and onto the young plant of 1.5 leaf stage was sprayed the desired compound (I) shown in Table 3 and Table 4, prepared into the wettable agent according to Example 3 or 8, diluted to 200 ppm with a water containing a surfactant (0.01 %) in each 20 ml per one pot.

After spraying, the plant was cultivated in a glass greenhouse for 2 days, and then a suspension of spores (7 x $10^4$ spores/ml) of barley powdery mildew microorganism (Eryziphe graminis ) prepared from afflicted leave was uniformly sprayed and inoculated on the plant.

After inoculation, the plant was grown in a glass greenhouse for one week, and the extent of barley powdery mildew lesion was examined.

Judgement of the chemical effect was compared with the extent of the lesion of the non-treated group.

The results are shown in Table 7 according to the same evaluation method as in Example 9. As Control compound, the compound (C) used in Example 9 was employed at 200 ppm.

Table 7

| Compound No. | Preventive effect against barley powdery mildew |
|---|---|
| 3 | 5 |
| 6 | 5 |
| 7 | 5 |
| 8 | 5 |
| 9 | 5 |
| 27 | 5 |
| 28 | 5 |
| 30 | 5 |
| 31 | 5 |
| C | 0 |

Example 12

[Control activity test against wheat red mould (preventive effect)]

In a plastic planting pot of 6 cm in diameter, 10 wheats (species: Kobushikomugi) were grown per one pot, and onto the young plant body of 1.5 leaf stage was sprayed the desired compound (I) shown in Table 3 and Table 4, prepared into the wettable agent according to Example 3 or 8, diluted to 200 ppm with a water containing a surfactant (0.01 %) in each 20 ml per one pot.

After spraying, the plant was cultivated in a glass greenhouse for 2 days, and a suspension of spores (7 x $10^4$ spores/ml) of wheat red mould microorganism (Puccinia dispersa ) was uniformly sprayed and inoculated on the plant.

After inoculation, the plant was grown in a glass greenhouse for one week, and the extent of barley powdery mildew lesion was examined.

Judgement of the chemical effect was compared with the extent of the lesion of the non-treated group.

The results are shown in Table 8 according to the same evaluation method as in Example 9. As Control compound, the compound (C) used in Example 9 was employed at 200 ppm.

Table 8

| Compound No. | Preventive effect against wheat red mould |
|---|---|
| 3 | 5 |
| 6 | 5 |
| 7 | 5 |
| 8 | 5 |
| 15 | 4 |
| 27 | 5 |
| 28 | 5 |
| 29 | 5 |
| 30 | 4 |
| 31 | 5 |
| C | 0 |

Example 13

[Control activity test against cucumber downy mildew (therapeutical effect)]

In a plastic planting pot of 6 cm in diameter, one cucumber (species: Sagami Hanpaku) was grown and onto the young plant of 1.5 leaf stage, a suspension of spores (7 x $10^4$ spores/ml) of cucumber downy mildew (Pseudoperonospora cubensis) was uniformly sprayed and inoculated.

After maintained at 20 °C in darkness for one day, the compound (I) shown in Table 3 and Table 4, prepared into a wettable agent according to Example 3 or 8, diluted to 500 ppm with a water containing a surfactant (0.01 %) was sprayed in an amount of each 20 ml per pot.

After spraying, the plant was cultivated in a glass greenhouse for 5 days and the extent of the cucumber downy mildew appeared in the first leave was examined. Judgement of the chemical effect was compared with the extent of the lesion of the non-treated group.

The results are shown in Table 9 according to the same evaluation method as in Example 9. As Control compound, the compound (C) shown in Example 9 was used at 500 ppm.

Table 9

| Compound No. | Therapeutical effect against cucumber downy mildew |
|---|---|
| 6 | 5 |
| 7 | 5 |
| 8 | 5 |
| 10 | 5 |
| C | 0 |

According to the present invention, oxime ether derivatives having excellent germicide effect can be provided.

## Claims

1. A compound represented by the formula (I):

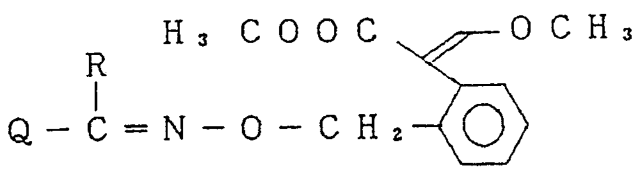

(I)

wherein R represents at least one selected from the group consisting of an alkylthio group having 1 to 10 carbon atoms, a halo-lower alkyl group and cyano group;
when R is an alkylthio group having 1 to 10 carbon atoms or a halo-lower alkyl group, Q represents at least one selected from the group consisting of a phenyl group substituted with an alkyl group having 1 to 6 carbon atoms, a halogen atom, nitro group, an alkoxy group having 1 to 4 carbon atoms, a phenoxy group which may be substituted, methylenedioxy group, cyano group or a halo-lower alkyl group, pyridyl group which may be substituted, a naphthyl group, a hetoroaryl group, cinnamyl group which may be substituted, and a cycloalkyl group having 3 to 6 carbon atoms; and
when R is cyano group, Q represents at least one selected from the group consisting of a phenyl group which may be substituted with an alkyl group having 1 to 6 carbon atoms, a halogen atom, nitro group, an alkoxy group having 1 to 4 carbon atoms, a phenoxy group which may be substituted, methylenedioxy group, cyano group or a halo-lower alkyl group, a naphthyl group, cinnamyl group which may be

substituted, and a cycloalkyl group having 3 to 6 carbon atoms.

2. The compound according to Claim 1, wherein the substituent in the phenyl group which may be substituted is stranght- or branched-chain alkyl group having 1 to 4 carbon atoms.

3. The compound according to Claim 2, wherein the substituent in the phenyl group which may be substituted is at least one of methyl group, ethyl group and t-butyl group.

4. The compound according to Claim 1, wherein the substituent in the phenyl group which may be substituted is nitro group at 2-, 3- or 4-position.

5. The compound according to Claim 1, wherein the substituent in the phenyl group which may be substituted is chlorine atom, bromine atom or iodine atom.

6. The compound according to Claim 5, wherein the substituent in the phenyl group which may be substituted is chlorine atom, bromine atom or iodine atom at 2-, 3- or 4-position.

7. The compound according to Claim 6, wherein the substituent in the phenyl group which may be substituted is a straight- or branched-chain alkoxy group having 1 to 4 carbon atoms.

8. The compound according to Claim 7, wherein the substituent in the phenyl group which may be substituted is at least one of methoxy group, ethoxy group, propoxy group and butoxy group.

9. The compound according to Claim 8, wherein the substituent in the phenyl group which may be substituted is at least one of methoxy group, ethoxy group, propoxy group and butoxy group at 2-position.

10. The compound according to Claim 1, wherein the substituent in the phenyl group which may be substituted is at least one of chloromethyl group, fluoromethyl group, trifluoromethyl group, 1- or 2-chloroethyl group and 1- or 2-fluoroethyl group.

11. The compound according-to Claim 1, wherein the substituent in the phenyl group which may be substituted is a halogen atom at 4-position.

12. The compound according to Claim 1, wherein the substituent in the phenyl group which may be substituted is cyano group at 3- or 4-position.

13. The compound according to Claim 1, wherein the compound represented by the formula (I) is a compound represented by the formula (I-A):

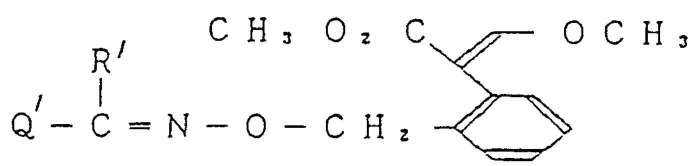

(I-A)

wherein Q′ represents a phenyl group substituted with a halogen atom, nitro group or an alkoxy group, naphthyl group, thiophen or a cyclopropyl group; R′ represents trifluoromethyl group.

14. The compound according to Claim 1, wherein the compound represented by the formula (I) is a compound represented by the formula (I-B):

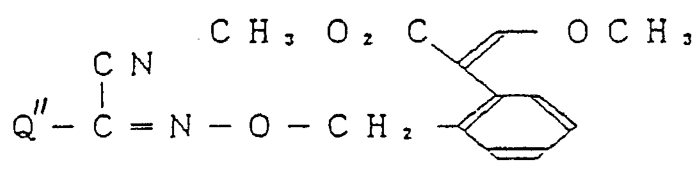

(I-B)

(wherein Q″ represents a phenyl group which may be substituted with an alkyl group having 1 to 6 carbon atoms, a halogen atom, nitro group, an alkoxy group having 1 to 4 carbon atoms or phenoxy group.

15. The compound according to Claim 1, wherein R represents an alkylthio group having 1 to 10 carbon atoms or a halo-lower alkyl group; and Q represents at least one selected from the group consisting of a phenyl group substituted with an alkyl group having 1 to 6 carbon atoms, a halogen atom, nitro group, an alkoxy group having 1 to 4 carbon atoms, a phenoxy group which may be substituted, methylenedioxy group, cyano group or a halo-lower alkyl group, pyridyl group which may be substituted, a naphthyl group, a hetoroaryl group, cinnamyl group which may be substituted, and a cycloalkyl group having 3 to 6 carbon atoms.

16. The compound according to Claim 15, wherein R is an alkylthio group and Q is at least one of as phenyl group substituted with at least one selected from the group consisting of mono halogen atom, mono

alkyl group having 1 to 6 carbon atoms and a branched alkyl group having 1 to 6 carbon atoms.

17. The compound according to Claim 15, wherein R is an alkylthio group and Q is a phenyl group substituted with a mono group.

18. The compound according to Claim 15, wherein R is at least one of chloromethyl group, fluoromethyl group, trifluoromethyl group, 1- or 2-chloroethyl group and 1- or 2-fluoroethyl group.

19. The compound according to Claim 1, wherein R is cyano group, and Q represents at least one selected from the group consisting of a phenyl group which may be substituted with an alkyl group having 1 to 6 carbon atoms, a halogen atom, nitro group, an alkoxy group having 1 to 4 carbon atoms, a phenoxy group which may be substituted, methylenedioxy group, cyano group or a halo-lower alkyl group, a naphthyl group, cinnamyl group which may be substituted, and a cycloalkyl group having 3 to 6 carbon atoms.

20. The compound according to Claim 18, wherein R is cyano group and Q is a phenyl group substituted with an alkyl group having 1 to 6 carbon atoms, a halogen atom, nitro group, an alkoxy group having 1 to 4 carbon atoms, a phenoxy group which may be substituted, methylenedioxy group, cyano group or a halo-lower alkyl group.

21. The compound according to Claim 18, wherein R is cyano group and Q is a phenyl group substituted with an alkyl group having 1 to 6 carbon atoms at 4-position or a halogen atom.

22. The compound according to Claim 18, wherein R is cyano group and Q is 3- or 4-trifluoromethylphenyl.

23. A method for preparing a compound represented by the formula (I) as defined in Claim 1, which comprises reacting a compound represented by the formula (II):

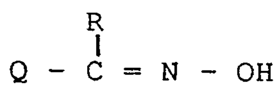

(II)

wherein Q and R are the same as defined in Claim 1, with a compound of the formula (III):

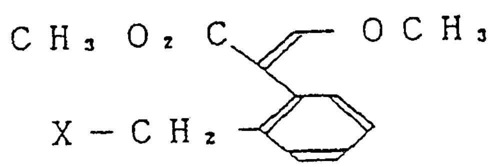

(III)

wherein X represents an eliminable group, under alkaline conditions.

24. The method for preparing a compound represented by the formula (I) according to Claim 22, wherein the compound represented by the formula (III) is represented by the formula (III-A):

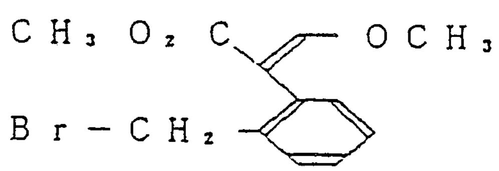

(III-A)

25. The method for preparing a compound represented by the formula (I) according to Claim 22, wherein the organic solvent is at least one selected from the group consisting of aprotic solvents, ether solvents and polar solvents.

26. The method for preparing a compound represented by the formula (I) according to Claim 22, wherein the organic solvent is at least one selected from the group consisting of acetonitrile, dimethylformamide, dimethyl sulfoxide, tetrahydrofuran, diethyl ether, dioxane and acetone.

27. The method for preparing a compound represented by the formula (I) according to Claim 22, wherein the base is at least one of potassium carbonate, sodium carbonate and sodium hydride.

28. The method for preparing a compound represented by the formula (I) according to Claim 22, wherein the eliminable group represented by X is at least one selected from the group consisting of a halogen atom, an alkanesulfonyloxy group which may be substituted with a halogen atom, and an arylsulfonyloxy group.

29. The method for preparing a compound represented by the formula (I) according to Claim 1, wherein the eliminable group represented by X is at least one selected from the group consisting of chlorine, bromine,

iodine, methane-sulfonyloxy, ethanesulfonyloxy, trifluoromethanesulfonyloxy, benzene-sulfonyloxy, p-toluenesulfonyloxy.

30. A germicide comprising a compound of the formula (I) as defined in Claim 1 as the active ingredient.

European Patent Office

# EUROPEAN SEARCH REPORT

Application Number

EP 90311946.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP - A1 - 0 370 629 (IMPERIAL CHEMICAL INDUSTRIES) * Claims 1,4 * -- | 1-29 | C 07 C 251/60<br>C 07 C 323/47<br>A 01 N 37/36 |
| A | GB - A - 1 601 348 (SHELL INTERNATIONALE RESEARCH) * Claims 1,9 * ---- | 1,23 | |
| | | | TECHNICAL FIELDS SEARCHED (Int. Cl.5) |
| | | | C 07 C 251/00<br>C 07 C 323/00 |

The present search report has been drawn up for all claims

| Place of search | Date of completion of the search | Examiner |
|---|---|---|
| VIENNA | 01-02-1991 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document